(19) Europäisches Patentamt
European Patent Office
Office européen des brevets

(11) Numéro de publication: **0 143 123 B1**

(12) # FASCICULE DE BREVET EUROPEEN

(45) Date de publication du fascicule du brevet: **25.07.90**

(21) Numéro de dépôt: **83201252.0**

(22) Date de dépôt: **19.05.81**

(60) Numéro de publication de la demande initiale en application de l' article 76 CBE: **0 040 573**

(51) Int. Cl.$^5$: **C 07 D 265/22,**
C 07 D 277/46,
C 07 D 263/48, C 07 D 233/88
// C07D413/12, C07D417/12,
C07D401/12, C07D215/56

(54) **Produits industriels nouveaux utilisés notamment comme intermédiaires dans la préparation de nouveaux dérivés du 4-hydroxy 3-quinoléine carboxamide substitués en 2 et la préparation desdits intermédiaires.**

(30) Priorité: **19.05.80 FR 8011100**

(43) Date de publication de la demande:
**05.06.85 Bulletin 85/23**

(45) Mention de la délivrance du brevet:
**25.07.90 Bulletin 90/30**

(84) Etats contractants désignés:
**AT BE CH DE FR GB IT LI LU NL SE**

(56) Documents cités:
**DE-A-2 241 012**

**BULLETIN OF THE CHEMICAL SOCIETY OF JAPAN, vol. 50, no. 8, aou7t 1977, pages 2164-2167, Tokyo, JP; N. ISHIKAWA et al.: "Preparation of perfluoroalkylated benzoheterocyclic compounds using hexafluoro-1,2-epoxypropane"**

**CHEMICAL ABSTRACTS, vol. 88, no. 3, 16 janvier 1978, page 628, no. 22820m, Columbus, Ohio, US; I. MASAYUKI et al.: "Synthetic studies on antiatherogenic agents. (III). Syntheses of carbamate derivative of quinazolines",**

(73) Titulaire: **ROUSSEL-UCLAF**
**35, boulevard des Invalides**
**F-75007 Paris (FR)**

(72) Inventeur: **Allais, André**
**1, Allée Eugénie**
**F-93220 Gagny (FR)**
Inventeur: **Clemence, François**
**2, rue Turgot**
**F-75009 Paris (FR)**
Inventeur: **Deraedt, Roger**
**23, Allée Jean-Baptiste Clément**
**F-93320 Pavillons-sous-Bois (FR)**
Inventeur: **Le Martret, Odile**
**42, Avenue de Versailles**
**F-75016 Paris (FR)**

(74) Mandataire: **Douetteau, Pierre et al**
**c/o ROUSSEL-UCLAF 102, route de Noisy Boite postale 9**
**F-93230 Romainville (FR)**

(56) Document cités:
**CHEMICAL ABSTRACTS, vol. 79, no. 11, 17 septembre 1973, page 186, no. 63208a, Columbus, Ohio, US; R. ALAZARD et al.: "Inactivation of alpha-chymotrypsin by a bifunctional reagent, 2-bromomethyl-3,1-benzoxazin-4-one"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 143 123 B1

## Description

La présente invention est une demande divisionnaire de la demande européenne publiée sous le numéro 0040573.

La présente invention concerne de nouveaux produits utilisés notamment comme intermédiaires dans la préparation de dérivés de la 4 hydroxy 3 quinoléine carboxamide substitués en 2 et leur procédé de préparation. Ces produits correspondent aux produits (VIII) et (X) de la demande européenne publiée sous le numéro 0040573 qui sont les produits permettant de préparer les produits de formule (I) thérapeutiquement actifs, décrits dans ladite demande européenne.

L'invention a pour objet les composés de formule (A) et de formule (B')

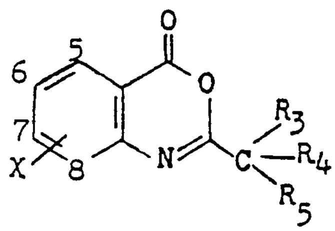 (A) 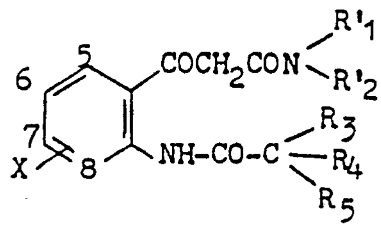 (B')

dans lesquelles X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy et dans lesquelles $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que:

a) $R_3$, $R_4$ et $R_5$ ne représentent pas en même temps chacun un atome de fluor.

b) L'un ou les deux de $R_3$ et $R_4$ ne représentent pas un atome d'halogène quand X représente un atome d'hydrogène, un atome d'halogène, un radical alcoyle renfermant de 1 à 5 atomes de carbone ou un radical alcoxy renfermant de 1 à 4 atomes de carbone.

c) X, $R_3$ et $R_4$ ne représentent pas en même temps un atome d'hydrogène quand $R_5$ représente un atome de brome et les composés de formule (B'), dans laquelle $R'_1$ représente un atome d'hydrogène ou un radical alcoyle renfermant de 1 à 4 atomes de carbone, $R'_2$ représente un atome d'hydrogène ou un radical choisi parmi les radicaux thiazolyle 4—5 dihydrothiazolyle, pyridinyle, thiényle, benzothiazolyle, oxazolyle, isoxazolyle, imidazolyle, pyrimidyle ou tétrazolyle et phényle, éventuellement substitué par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyle et g) le radical nitro, ou $R'_1$ et $R'_2$ forment ensemble l'un quelconque des cycles saturés ou insaturés mentionnés ci-dessus pour $R'_2$, ledit cycle étant alors relié à l'atome d'azote par une double liaison.

Lorsque $R'_1$ représente un radical alcoyle, il s'agit de préférence du radical méthyle ou éthyle.

Lorsque X représente un atome d'halogène, il s'agit de préférence d'un atome de chlore.

Lorsque X représente un radical alcoyle, il s'agit de préférence du radical méthyle, éthyle, n-propyle, n-butyle, n-pentyle, isopropyle ou isobutyle.

Lorsque X représente un radical alcoxy, il s'agit de préférence du radical méthoxy, éthoxy ou n-propoxy.

Lorsque $R'_2$ représente un cycle, il s'agit de préférence d'un cycle thiazolyle, phényle, pyridinyle, thiényle, benzothiazolyle, oxazolyle ou imidazolyle.

Lorsque $R'_2$ représente un cycle substitué, le ou les substituants sont de préférence choisis dans le groupe constitué par l'atome de chlore, le radical méthyle, le radical éthyle, le radical diméthylaminométhyle, le radical phényle, le radical méthoxy, le radical éthoxy, le radical hydroxy, le radical trifluorométhyle et le radical nitro.

Lorsque $R_3$ représente un radical alcoyle, il s'agit de préférence du radical méthyle ou éthyle.

Lorsque $R_3$ représente un atome d'halogène, il s'agit de préférence d'un atome de fluor, de chlore ou de brome.

$R_4$ représente de préférence un atome de fluor, de chlore ou de brome.

$R_5$ représente de préférence un atome de chlore ou de brome.

L'invention a notamment pour objet les composés tels que définis ci-dessus, répondant à la formule (B):

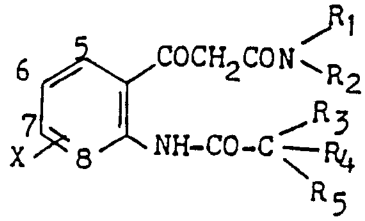

(B)

dans laquelle X est défini comme ci-dessus, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle ou tétrazolyle, éventuellement substitué par un radical alcoyle renfermant de 1 à 4 atomes de carbone, et phényle, éventuellement substitué par au moins un radical choisi dans le groupe formé par les radicaux hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène et $R_3$, $R_4$ et $R_5$ sont définis comme ci-dessus.

L'invention a plus particulièrement pour objet les composés de formule (A) ou (B), tels que définis ci-dessus pour lesquels $R_3$ représente un atome d'hydrogène, un radical alcoyle ou le même atome d'halogène que $R_5$, $R_4$ représente un atome d'hydrogène ou le même atome d'halogène que $R_5$.

L'invention a notamment pour objet les composés de formule (A) ou (B), tels que définis ci-dessus pour lesquels X représente un radical trifluorométhyle et les composés de formule (B) tels que définis ci-dessus pour lesquels $R_1$ représente un atome d'hydrogène.

L'invention a notamment pour objet les composés de formule (A) et (B) pour lesquels $R_3$ et $R_4$, identiques ou différents, représentent un atome de chlore, et plus particulièrement parmi ceux-ci, les composés pour lesquels le radical

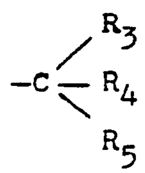

représente le radical $-CHCl_2$.

Parmi les composés de l'invention, ou peut citer tout particulièrement les composés de formule (B) tels que définis ci-dessus pour lesquels $R_2$ représente le radical thiazolyle.

L'invention a pour objet un procédé de préparation des composés de formules (A) et (B'), telles que définies ci-dessus caractérisé en ce que l'on soumet un composé de formule (VI):

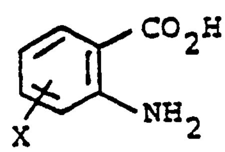

(VI)

dans laquelle X conserve sa signification précédente, à l'action d'un acide de formule (VII):

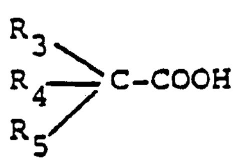

(VII)

dans laquelle $R_3$, $R_4$, $R_5$ conservant leurs significations précédentes ou d'un dérivé fonctionnel de l'acide de formule (VII), pour obtenir un composé de formule (A):

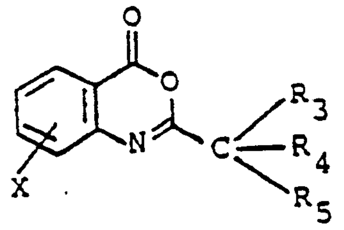

(A)

dans laquelle X conserve sa signification précédente, que l'on soumet à l'action d'un composé de formule (IX):

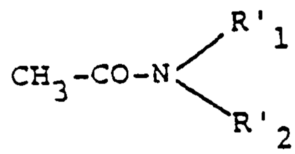

(IX)

dans laquelle $R'_1$ et $R'_2$ conservent leurs significations précédentes, pour obtenir le composé de formule (B'):

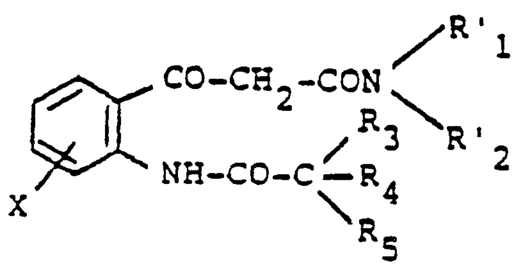

(B')

dans laquelle $R'_1$, $R'_2$, $R_3$, $R_4$ et $R_5$ conservent leurs significations précédentes.

Dans un mode de réalisation préférée du procédé de l'invention:

— le dérivé fonctionnel de l'acide de formule (VII) est un halogénure ou un anhydride;

— la réaction entre le composé de formule (A) et le composé de formule (B') a lieu en présence d'un organolithien ou d'un amidure de lithium, par exemple en présence de butyllithium ou de diisoproylamidure de lithium;

— la cyclisation du composé de formule (B') est effectuée en présence d'un agent alcalin, tel qu'un hydrure ou un carbonate alcalin, ou une amine, par exemple en présence d'hydrure de sodium, de carbonate de sodium ou de potassium, de pipéridine, de 4-aminopyridine, de triéthylamine, de 1,5-diazabicyclo /4,30/ non-5-ène, de 1,4-diazabicyclo /2,2,2/ octane ou de 1,5-diazabicyclo /5,4,0/ undéc-5-ène.

Les composés de formule (VI) utilisés comme produits de départ du procédé de l'invention sont des produits connus d'une façon générale, qui peuvent être préparés selon les procédés indiqués dans la demande de brevet français no 2 340 735 ou dans le brevet français no 2 157 874.

Les exemples suivants illustrent l'invention sans toutefois la limiter.

## Exemple 1

(A): 2-dichlorométhyl 8-trifluorométhyl 4H-3,1-benzoxazin-4-one.

On chauffe progressivement sous agitation 17,44 g d'acide 2-amino 3-(trifluorométhyl) benzoïque préparé selon le procédé indiqué dans J. Med. Chem. 16 (2) 101, 6 (1973) et 34,63 g de chlorure de dichloroacétyle. Aux alentours de 50°C, on observe une prise en masse, ainsi qu'un dégagement rapide et régulier d'HCl qui se poursuit pendant 1 heure. On observe alors vers 100°C une fluidification du milieu réactionnel, une intensification du dégagement d'HCl et une élévation de la température jusqu'à 127°C. On poursuit le chauffage jusqu'à cessation du dégagement d'HCl et l'on obtient une solution brune. On refroidit dans un bain d'eau glacée tout en agitant. On essore le précipité formé, empâte dans l'éther, puis dans le méthanol. On lave au méthanol. On obtient 23,82 g du produit recherché fondant à 179°C.

## Exemple 2

(B'): 2-(dichloroacétylamino) béta-oxo-N-(2-thiazolyl) 3-trifluorométhyl benzène propanamide.

On ajoute entre −3° et −1°C sous agitation et sous azote, 87,7 $cm^3$ d'une solution de n-butyllithium dans l'hexane titrant 1,1 moles par litre, dans une solution renfermant 8,65 g de N-(2-thiazolyl) acétamide et

304 $cm^3$ de tetrahydrofurane. On maintient encore 20 minutes sous agitation entre −3 et −1°C. On refroidit à −75°C, et l'on ajoute à cette température sous bonne agitation entre −73 et −75°C, 9,073 g de 2-dichlorométhyl 8-trifluorométhyl 4H-3,1-benzoxazin-4-one en solution dans 95,5 cc de tétrahydrofurane. On agite à −75°C pendant 2 heures 30 la solution brune obtenue. On verse sur un mélange d'eau, de glace et d'HCl. On décante et essore l'insoluble. On obtient 935 mg du produit recherché. F = 223°C. On extrait les phases aqueuses à l'acétate d'éthyle. On lave à l'eau, sèche et évapore à sec. On obtient ainsi 15,2 g du produit brut recherché. On y ajoute 100 cc de chlorure de méthylène, triture, essore l'insoluble, empâte dans 30 cc de chlorure de méthylène, essore, lave avec 10 cc de chlorure de méthylène. On obtient 7,47 g du produit recherché fondant à 223°C.

### Exemple 3

(A): 2-chlorométhyl 8-trifluorométhyl 4H-3,1-benzoxazin-3-one.

En opérant comme au Stade A de l'exemple 12, à partir d'acide 2-amino 3-trifluorométhyl benzoïque et de chlorure de chloracétyle, on a obtenu le produit recherché fondant à 76 ≃ 78°C.

### Exemple 4

(B'): 2-(chloroacétylamino) béta-oxo N-(2-thiazolyl) 3-trifluorométhyl benzène propanamide.

En opérant comme au Stade B de l'exemple 12, à partir du produit préparé au stade A du présent exemple et de N-(2-thiazolyl) acétamide, on obtient le produit recherché fondant à 192 ≃ 194°C.

### Exemple 5

(A): 2-(1-chloroéthyl) 8-trifluorométhyl 4H-3,1-benzoxazin-4-one.

En opérant comme au stade A de l'exemple 12, à partir du chlorure de 1-chloropropionyle et de l'acide 2-amino 3-trifluorométhyl benzoïque, on a obtenu le produit recherché. F = 112°C.

### Exemple 6

(B'): 2-(1-chloropropionylamino) béta-oxo 3-trifluorométhyl N-(2-thiazolyl) benzène propanamide.

En opérant comme au stade B de l'exemple 12, à partir du produit obtenu au stade A ci-dessus et de N-(2-thiazolyl) acétamide, on a obtenu le produit recherché. F = 215°C.

### Exemple 7

(A): 2-(1,1-dichloroéthyl) 8-trifluorométhyl 4H-3,1-benzoxazin-4-one.

En opérant comme au stade A de l'exemple 12, à partir d'acide 2-amino 3-trifluorométhyl benzoïque et de chlorure de dichloropropionyle, on a obtenu le produit recherché. F = 120°C.

### Exemple 8

(B'): 2-(1,1-dichloropropionylamino) béta-oxo N-(2-thiazolyl) 3-trifluorométhyl benzène propanamide.

En opérant comme au stade B de l'exemple 12, à partir du produit obtenu au stade A ci-dessus et de N-(2-thiazolyl) acétamide, on a obtenu le produit recherché. F = 136—138°C.

### Exemple 9

(A): 2-(difluorométhyl) 8-trifluorométhyl 4H-3,1-benzoxazin-4-one.

On opère comme au stade A de l'exemple 12, à partir d'acide 2-amino 3-trifluorométhyl benzoïque et d'anhydride de l'acide difluoroacétique et obtient le produit attendu. F = 82°C.

### Exemple 10

(B'): 2-(difluoroacétylamino béta-oxo 3-trifluorométhyl N-(2-thiazolyl) benzène propanamide.

On opère comme au stade B de l'exemple 12, à partir du produit obtenu au stade A ci-dessus et de N-(2-thiazolyl) acétamide, et obtient le produit attendu. F = 206°C.

### Exemple 11

(B'): 2-(dichloroacétylamino) béta-oxo N-(2-oxazolyl) 3-trifluorométhyl benzène propanamide.

En opérant comme au stade B de l'exemple 12, à partir du produit préparé au stade A de l'exemple 12, et du N-(2-oxazolyl) acétamide, on obtient le produit recherché.

### Exemple 12

(B'): 2-(dichloroacétylamino) béta-oxo N-(1-méthyl 2-imidazolyl) 3-trifluorométhyl benzène propanamide.

En opérant comme au stade B de l'exemple 12, à partir du produit préparé au stade A de l'exemple 12 et du N-1-méthyl 2-imidazolyl) acétamide, on obtient le produit recherché.

### Exemples de compositions pharmaceutiques

On a préparé des comprimés répondant à la formule suivante:

| | |
|---|---|
| Produit de l'exemple 1 | 50 mg |
| Excipient q.s. pour un comprimé terminé à | 350 mg |

(Détail de l'excipient: lactose, talc, amidon, stéarate de magnésium).

## Revendications

1. Les composés de formule (A) et de formule (B'):

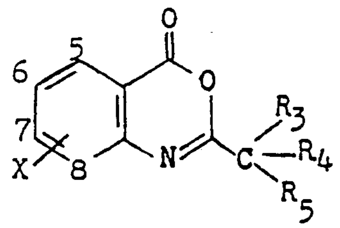 (A)

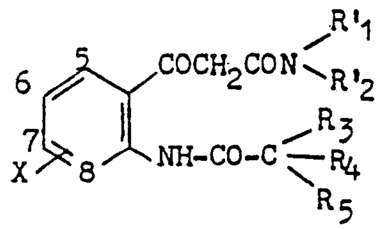 (B')

dans lesquelles X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy et dans lesquelles $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que:

a) $R_3$, $R_4$ et $R_5$ ne représentent pas en même temps chacun un atome de fluor.

b) L'un ou les deux de $R_3$ et $R_4$ ne représentant pas un atome d'halogène quand X représente un atome d'hydrogène, un atome d'halogène, un radical alcoyle renfermant de 1 à 5 atomes de carbone ou un radical alcoxy renfermant de 1 à 4 atomes de carbone.

c) X, $R_3$ et $R_4$ ne représentent pas en même temps un atome d'hydrogène quand $R_5$ représente un atome de brome et les composés de formule (B'), dans laquelle $R'_1$ représente un atome d'hydrogène ou un radical alcoyle renfermant de 1 à 4 atomes de carbone, $R'_2$ représente un atome d'hydrogène ou un radical choisi parmi les radicaux thiazolyle 4—5 dihydrothiazolyle, pyridinyle, thiènyle, benzothiazolyle, oxazolyle, isoxazolyle, imidazolyle, pyrimidyle ou tétrazolyle et phényle, éventuellement substitué par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyle et g) le radical nitro, ou $R'_1$ et $R'_2$ forment ensemble l'un quelconque des cycles saturés ou insaturés mentionnés ci-dessus pour $R'_2$, ledit cycle étant alors relié à l'atome d'azote par une double liaison.

2. Les composés selon la revendication 1, répondant à la formule (B)

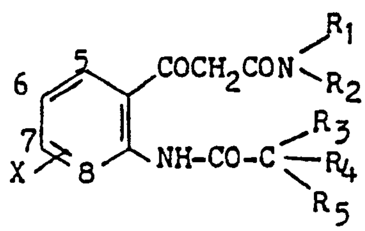 (B)

dans laquelle X est défini comme à la revendication 1, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle ou tétrazolyle, éventuellement substitué par un radical alcoyle renfermant de 1 à 4 atomes de carbone, et phényle, éventuellement substitué par au moins un radical choisi dans le groupe formé par les radicaux hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène et $R_3$, $R_4$ et $R_5$ sont définis comme à la revendication 1.

3. Les composés de formule (A) ou (B), tels que définis aux revendications 1 ou 2, pour lesquels $R_3$ représente un atome d'hydrogène, un radical alcoyle ou le même atome d'halogène que $R_5$, $R_4$ représente un atome d'hydrogène ou le même atome d'halogène que $R_5$.

4. Les composés de formule (A) ou (B), tels que définis à l'une quelconque des revendications 1 à 3, pour lesquels X représente un radical trifluorométhyle.

5. Les composés de formule (B) tels que définis à l'une quelconque des revendications 2 à 4, pour lesquels $R_1$ représente un atome d'hydrogène.

6. Les composés de formule (A) ou (B) tels que définis à l'une quelconque des revendications 1 à 5, pour lesquels $R_3$ et $R_4$ identiques ou différents, représentent un atome d'hydrogène ou un atomes de chlore, et $R_5$ représente un atome de chlore.

7. Les composés de formule (A) ou (B) tels que définis à la revendication 6 pour lesquels le radical

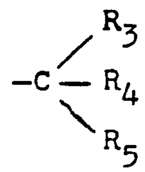

représente le radical $—CHCl_2$.

8. Les composés de formule (B) tels que définis à l'une quelconque des revendications 2 à 7, pour lesquels $R_2$ représente le radical thiazolyle.

9. Procédé de préparation des composés de formules (A) et (B'), telles que définies à la revendication 1, caractérisé en ce que l'on soumet un composé de formule (VI):

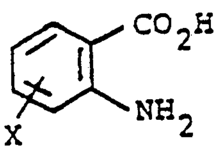

(VI)

dans laquelle X conserve sa signification donnée à la revendication 1, à l'action d'un acide de formule (VII)

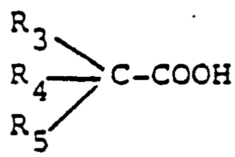

(VII)

dans laquelle $R_3$, $R_4$, $R_5$ conservent leurs significations données à la revendication 1 ou d'un dérivé fonctionnel de l'acide de formule (VII), pour obtenir un composé de formule (A)

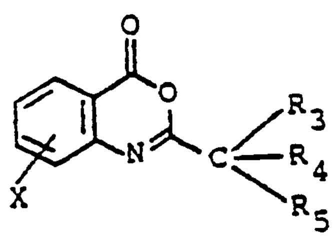

(A)

dans laquelle X conserve sa signification précédente, que l'on soumet, le cas échéant, à l'action d'un composé de formule (IX):

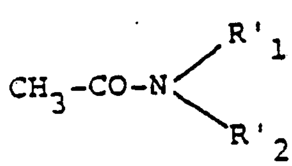

(IX)

dans laquelle $R'_1$ et $R'_2$ conservent leur significations données à la revendication 1, pour obtenir le composé de formule (B')

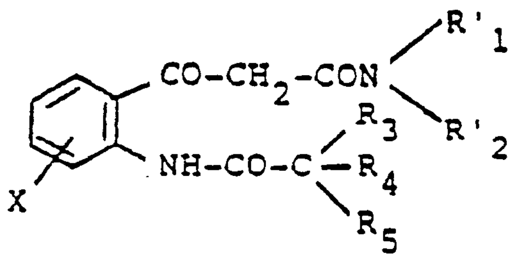 (B')

dans laquelle $R'_1$, $R'_2$, $R_3$, $R_4$ et $R_5$ conservent leurs significations précédentes.

10. Procédé selon la revendication 9, caractérisé en ce que

— le dérivé fonctionnel de l'acide de formule (VII) est un halogénure ou un anhydride,

— la réaction entre le composé de formule (A) et le composé de formule (IX) a lieu en présence d'un organolithien ou d'un amidure de lithium.

## Patentansprüche

1. Verbindungen der Formel (A) und der Formel (B')

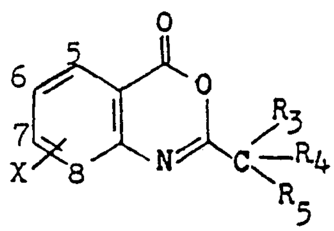 (A) 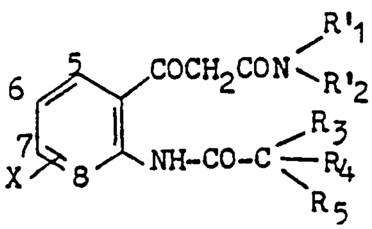 (B')

worin X in 5-, 6-, 7- oder 8-Stellung ein Wasserstoffatom, ein Halogenatom, eine lineare oder verzweigte Alkylgruppe mit 1 bis 5 Kohlenstoffatomen, eine lineare oder verzweigte Alkoxygruppe mit 1 bis 4 Kohlenstoffatomen, eine Trifluormethylgruppe, eine Trifluormethylthiogruppe oder eine Trifluormethoxygruppe bedeutet und worin $R_3$ für ein Wasserstoffatom, ein Halogenatom, eine Alkylgruppe mit 1 bis 4 Kohlenstoffatomen steht, $R_4$ für ein Wasserstoffatom oder ein Halogenatom steht und $R_5$ ein Halogenatom bedeutet, mit der Maßgabe, daß

(a) $R_3$, $R_4$ und $R_5$ nicht gleichzeitig ein Fluoratom bedeuten,

(b) einer oder beide Reste $R_3$ und $R_4$ kein Halogenatom darstellen, wenn X für ein Wasserstoffatom, ein Halogenatom, einen Alkylrest mit 1 bis 5 Kohlenstoffatomen oder einen Alkoxyrest mit 1 bis 4 Kohlenstoffatomen steht,

(c) X, $R_3$ und $R_4$ nicht gleichzeitig ein Wasserstoffatom bedeuten, wenn $R_5$ ein Bromatom wiedergibt, und die Verbindungen der Formel (B'), worin $R'_1$ für ein Wasserstoffatom oder eine Alkylgruppe mit 1 bis 4 Kohlenstoffatomen steht, $R'_2$ für ein Wasserstoffatom oder einen Rest steht, ausgewählt unter den Thiazolyl-, 4,5-Dihydrothiazolyl-, Pyridinyl-, Thienyl-, Benzothiazolyl-, Oxazolyl-, Isoxazolyl-, Imidazolyl-, Pyrimidyl- oder Tetrazolylresten und Phenyl, gegebenenfalls substituiert durch einen oder mehrere Reste, ausgewählt unter (a) den Halogenen, (b) den Alkylresten mit 1 bis 4 Kohlenstoffatomen, die unsubstituiert oder substituiert sind durch eine Amino-, Alkylamino- oder Dialkylaminogruppe, deren Alkylreste 1 bis 3 Kohlenstoffatome enthalten, (c) der Phenylgruppe, (d) den Alkoxyresten mit 1 bis 4 Kohlenstoffatomen, (e) der Hydroxygruppe, (f) dem Trifluormethylrest und (g) der Nitrogruppe, oder $R'_1$ und $R'_2$ gemeinsam einen der vorstehend für $R'_2$ erwähnten, gesättigten oder ungesättigten Ringe bilden, wobei dieser Ring dann an das Stickstoffatom durch eine Doppelbindung gebunden ist.

2. Verbindungen gemäß Anspruch 1 der Formel (B)

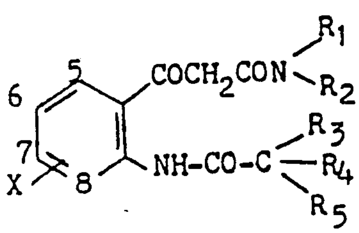 (B)

worin X wie in Anspruch 1 definiert ist, $R_1$ für ein Wasserstoffatom oder eine Alkylgruppe mit 1 bis 4

Kohlenstoffatomen steht, $R_2$ einen Rest bedeutet, ausgewählt unter den Thiazolyl-, 4,5-Dihydrothiazolyl-, Pyridinyl-, Oxazolyl-, Isoxazolyl-, Imidazolyl-, Pyrimidyl- oder Tetrazolylresten, gegebenenfalls substituiert durch eine Alkylgruppe mit 1 bis 4 Kohlenstoffatomen, und Phenyl, gegebenenfalls substituiert durch zumindest einen Rest, ausgewählt unter den Hydroxyresten, den Alkylresten mit 1 bis 4 Kohlenstoffatomen, den Alkoxyresten mit 1 bis 4 Kohlenstoffatomen, der Trifluormethylgruppe, der Nitrogruppe und den Halogenatomen, und $R_3$, $R_4$ und $R_5$ wie in Anspruch 1 definiert sind.

3. Verbindungen der Formel (A) oder (B) gemäß den Ansprüchen 1 oder 2, worin $R_3$ für ein Wasserstoffatom, eine Alkylgruppe oder das gleiche Halogenatom wie $R_5$ steht, $R_4$ ein Wasserstoffatom oder das gleiche Halogenatom wie $R_5$ wiedergibt.

4. Verbindungen der Formel (A) oder (B) gemäß einem der Ansprüche 1 bis 3, worin X für eine Trifluormethylgruppe steht.

5. Verbindungen der Formel (B) gemäß einem der Ansprüche 2 bis 4, worin $R_1$ für ein Wasserstoffatom steht.

6. Verbindungen der Formel (A) oder (B) gemäß einem der Ansprüche 1 bis 5, worin $R_3$ und $R_4$, die gleich oder voneinander verschieden sind, für ein Wasserstoffatom oder ein Chloratom stehen und $R_5$ ein Chloratom bedeutet.

7. Verbindungen der Formel (A) oder (B) gemäß Anspruch 6, worin der Rest

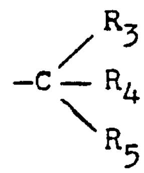

den Rest $-CHCl_2$ wiedergibt.

8. Verbindungen der Formel (B) gemäß einem der Ansprüche 2 bis 7, worin $R_2$ für den Thiazolylrest steht.

9. Verfahren zur Herstellung der Verbindungen der Formeln (A) und (B') gemäß Anspruch 1, dadurch gekennzeichnet, daß man eine Verbindung der Formel (VI)

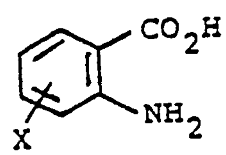

(VI)

worin X die in Anspruch 1 angegebene Bedentung besitzt, der Einwirkung einer Säure der Formel (VII)

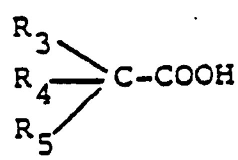

(VII)

worin $R_3$, $R_4$ und $R_5$ die in Anspruch 1 angegebenen Bedeutungen besitzen, oder eines funktionellen Derivats der Säure der Formel (VII) unterzieht, um zu einer Verbindung der Formel (A)

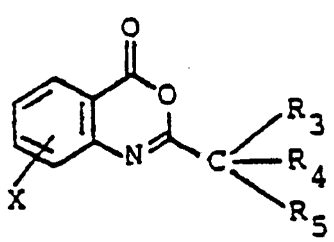

(A)

zu gelangen, worin X die vorstehende Bedeutung besitzt, die man der Einwirkung einer Verbindung der Formel (IX)

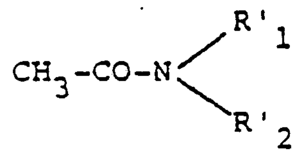

(IX)

worin $R'_1$ und $R'_2$ die in Anspruch 1 angegebenen Bedeutung besitzen, unterzieht, um zu der Verbindung der Formel (B')

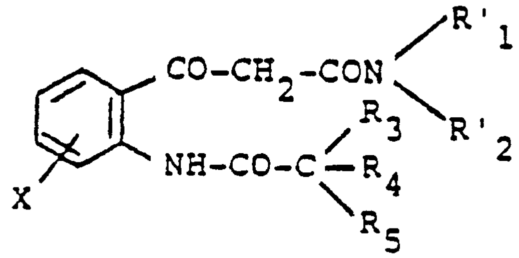

(B')

zu gelangen, worin $R'_1$, $R'_2$, $R_3$, $R_4$ und $R_5$ die vorstehenden Bedeutungen besitzen.

10. Verfahren gemäß Anspruch 9, dadurch gekennzeichnet, daß

das funktionelle Säurederivat der Formel (VII) ein Halogenid oder ein Anhydrid ist,

die Umsetzung zwischen der Verbindung der Formel (A) und der Verbindung der Formel (IX) in Anwesenheit einer Organolithiumverbindung oder eines Lithiumamids stattfindet.

**Claims**

1. The compounds of formula (A) and of formula (B'):

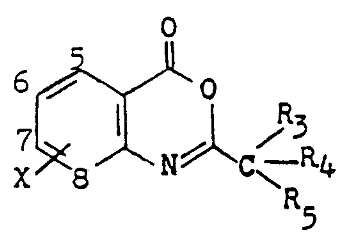

(A)

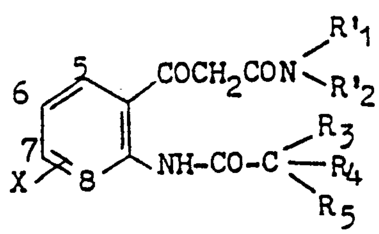

(B')

in which X in position 5, 6, 7 or 8, represents a hydrogen atom, a halogen atom, a linear or branched alkyl radical containing 1 to 5 carbon atoms, a linear or branched alkoxy radical containing 1 to 4 carbon atoms, a trifluoromethyl radical, a trifluoromethylthio radical or a trifluoromethoxy radical and in which $R_3$ represents a hydrogen atom, a halogen atom, an alkyl radical containing 1 to 4 carbon atoms, $R_4$ represents a hydrogen atom or a halogen atom, $R_5$ represents a halogen atom, on condition that:

a) $R_3$, $R_4$ and $R_5$ do not each represent, at the same time, a fluorine atom.

b) One of $R_3$ and $R_4$ does not represent a halogen atom when X represents a hydrogen atom, a halogen atom, an alkyl radical containing 1 to 5 carbon atoms or an alkoxy radical containing 1 to 4 carbon atoms.

c) X, $R_3$ and $R_4$ do not represent, at the same time, a hydrogen atom, when $R_5$ represents a bromine atom, and the compounds of formula (B'), in which $R'_1$ represents a hydrogen atom or an alkyl radical containing 1 to 4 carbon atoms, $R'_2$ represents a hydrogen atom or a radical chosen from the following radicals: thiazolyl, 4,5-dihydrothiazolyl, pyridinyl, thienyl, benzothiazolyl, oxazolyl, isoxazolyl, imidazolyl, pyrimidyl or tetrazolyl and phenyl, optionally substituted by one or more radicals chosen from the group constituted by a) the halogens, b) the alkyl radicals containing 1 to 4 carbon atoms, not substituted or substituted by an amino, alkylamino or dialkylamino radical the alkyl radicals of which contain 1 to 3 carbon atoms, c) the phenyl radical, d) the alkoxy radicals containing 1 to 4 carbon atoms, e) the hydroxy radical, f) the trifluoromethyl radical and g) the nitro radical, or $R'_1$ and $R'_2$ together form any one of the saturated or unsaturated rings mentioned above for $R'_2$, the said ring then being linked to a nitrogen atom by a double bond.

2. The compounds according to claim 1, corresponding to the formula (B):

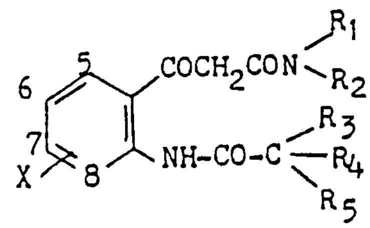

(B)

in which X is defined as in claim 1, $R_1$ represents a hydrogen atom or an alkyl radical containing 1 to 4 carbon atoms, $R_2$ represents a radical chosen from the following radicals: thiazolyl, 4,5-dihydrothiazolyl, pyridinyl, oxazolyl, isoxazolyl, imidazolyl, pyrimidyl or tetrazolyl, optionally substituted by an alkyl radical containing 1 to 4 carbon atoms, and phenyl, optionally substituted by at least one radical chosen from the group formed by the hydroxy radicals, the alkyl radicals containing 1 to 4 carbon atoms, the alkoxy radicals containing 1 to 4 carbon atoms, the trifluoromethyl radical, the nitro radical and the halogen atoms and $R_3$, $R_4$ and $R_5$ are defined as in claim 1.

3. The compounds of formula (A) or (B), as defined in claims 1 or 2, in which $R_3$ represents a hydrogen atom, an alkyl radical or the same halogen atom as $R_5$, $R_4$ represents a hydrogen atom or the same halogen atom as $R_5$.

4. The compounds of formula (A) or (B), as defined in any one of claims 1 to 3, in which X represents a trifluoromethyl radical.

5. The compounds of formula (B) as defined in any one of claims 2 to 4, in which $R_1$ represents a hydrogen atom.

6. The compounds of formula (A) or (B) as defined in any one of claims 1 to 5, in which $R_3$ and $R_4$, identical or different, represents a hydrogen atom or a chlorine atom, and $R_5$ represents a chlorine atom.

7. The compounds of formula (A) or (B) as defined in claim 6 in which the radical

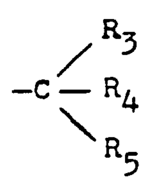

represents the radical $-CHCl_2$.

8. The compounds of formula (B) as defined in any one of claims 2 to 7, in which $R_2$ represents the thiazolyl radical.

9. Preparation process for the compounds of formulae (A) and (B') as defined in claim 1, characterized in that a compound of formula (VI):

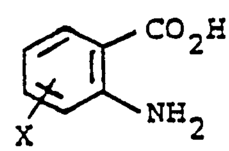

(VI)

in which X keeps its meaning given in claim 1, is subjected to the action of an acid of formula (VII):

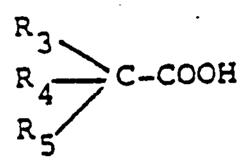

(VII)

in which $R_3$, $R_4$, $R_5$ keep their meanings given in claim 1, or of a functional derivative of the acid of formula (VII), so as to obtain a compound of formula (A):

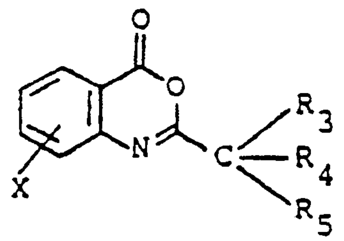

(A)

in which X keeps its previous meaning, which is subjected, if appropriate, to the action of a compound of formula (IX):

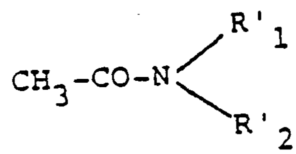

(IX)

in which $R'_1$ and $R'_2$ keep their meanings given in claim 1, so as to obtain the compound of formula (B'):

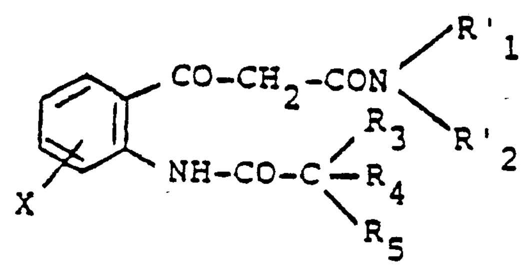

(B')

in which $R'_1$, $R'_2$, $R_3$, $R_4$ and $R_5$ keep their previous meanings.

10. Process according to claim 9, characterized in that

— the functional derivative of the acid of formula (VII) is a halide or an anhydride,

— the reaction between the compound of formula (A) and the compound of formula (IX) takes place in the presence of an organo-lithium compound or of a lithium amide.